# EUROPEAN PATENT SPECIFICATION

(11) **EP 1 224 128 B1**
(45) Date of publication and mention of the grant of the patent: **18.10.2006**
(21) Application number: 00955845.3
(22) Date of filing: 22.08.2000
(51) Int. Cl.: A47G 21/18, B65D 25/08

(54) **DISPENSING TUBE**
SPENDERTUBE
TUBE DISTRIBUTEUR

(30) Priority: 01.09.1999 US 387947
(43) Date of publication of application: 24.07.2002
(60) Divisional application: 06117621.0
(73) Proprietor: BIOGAIA AB, 103 64 Stockholm (SE)
(72) Inventor: THORBALL, Jorgen, DK-2830 Virum (DK); SKOLLING, Otto, S-187 50 Täby (SE); CASAS, Ivan, A., Raleigh, NC 27612 (US)
(74) Representative: Fagerlin, Heléne
(86) International application number: PCT/US2000/023065
(87) International publication number: WO 2001/015985

(56) References cited:
- GB-A- 512 831
- US-A- 1 996 203
- US-A- 3 615 595
- US-A- 3 717 476
- US-A- 4 816 268
- US-A- 5 190 755
- US-A- 5 334 348
- US-A- 5 681 564
- US-A- 5 718 681
- US-A- 5 820 023
- US-A- 5 921 955

## Description

### BACKGROUND OF THE INVENTION

### Field of the Invention

This invention relates to devices for dispensing living cells of a probiotic microorganism into a liquid, such as beverages, and a method for making the device.

### Description of the Related Art

In the pharmaceutical and food industry it is well-known that addition of health-promoting bacteria (e.g. probiotic bacteria such as lactic bacteria or bifido bacteria) allow people to maintain a proper gut function. However, it has been difficult and relatively expensive to have an acceptable shelf life of a mixed product that contains these bacteria. The problem has been that such drinks or enteral solutions go through a thermal sterilization or are aseptically filled in presterilized containers, thus killing or removing any live bacteria added during the production process. If the bacteria are added directly into the solution during the production/filling process and after sterilization, the bacteria are likely to be re-activated by the presence of water, and would accordingly multiply and finally die within a few weeks or months after production. The metabolites of the bacteria might also change the solution taste and nutritional value.

To avoid the interaction between the solution and the bacteria prior to ingestion, special delivery systems have been integrated into solution containers like e.g., Tetrabrik or Pet bottles (see, for example, co-pending PCT application PCT/US98/21490). Since these delivery systems are more or less an integral part of the packaging, the producer cannot choose during or after production to have some of the products have the delivery system and some not to have it.

Attempts to solve these problems include the use of tubular devices, such as telescopic packaging infusion units formed as tubes from a liquid impermeable material. For example U.S. Pat. No. 3,102,465 and PCT/AAU97/00680 disclose straw-shaped units that can be opened so that the ingredient contained in the unit can be dispensed. A number of patents, for example, U.S. Pat. Nos. 4,860,929 and 4,986,451, provide tubular devices closed on both ends and having perforations along the sides to allow granular material to be released and dissolved in contact with water or another solvent. Other methods of adding a material to a liquid by means of a straw-device include coating the outside of one end of a straw with a flavored coating that dissolves when the straw is placed in a liquid or making the end of a straw in the form of a spoon made of a soluble substance. Other straw-shaped novelty inventions provide straws with internal or external decorative features and substances.

It is therefore an object of the invention to provide a simple low-cost and consumer-friendly system to protect bacteria for an extended term at room temperature, and have a ready-to-use system for the patient or the user after this extended term.

It is a further object to provide a device that enable addition to drinks of living cells of a probiotic micro-organism, using a straw that the consumer can then use to sip the drink.

It is a further object of the invention to provide a means of adding living cells of a probiotic bacteria or other additives to beverages such as dairy products or soft drinks or to enteral solutions which have been through an aseptic or sterile treatment, e.g., sterile filtration, irradiation or thermal sterilization.

It is a further object of the invention to provide a device for adding living cells of a probiotic micro organism to beverages which has a water and moisture tight container until it is opened and ready for use.

It is a further object of the invention to provide a means for long-term storage of health promoting bacteria.

It is a further object of the invention to provide a new delivery system for other moisture-sensitive or oxygen-sensitive components, such as certain amino acids, peptides, nucleotides, vitamins, hormones and proteins.

Other objects and advantages will be more fully apparent from the following disclosure and appended claims.

### US-A-5 921 955

This reference is for an apparatus for adding a beneficial agent to a liquid for drinking, in which the beneficial agent is placed in a retaining pocket defined in a support structure.

### US-A-4 816 268

The reference is for a device for dispensing a comestible product having a "porous lattice structure" into a liquid and a method for preparing such device comprising dissolving a comestible product in a suitable solvent and crystallizing the product in a dispensing means.

### GB-A-512 831

This reference is for an invention related to tubes for drinking purposes and have a dry coating on their inner wall of some soluble material for flavouring the liquid drawn up the tube.

### US-A-3 717476

Harvey teaches a container construction comprising a straw sealed at both ends and means for breaking the seals without removing the straw from the container, stating that the construction is particularly useful in conjunction with a straw coated on the interior surface with a flavouring or taste-modifying material (for example, column 3, lines 10-45).

### US-A-3 615 595

Guttag teaches a tubular drinking straw having at least a portion of an exposed wall formed of a water insoluble hydrophilic acrylate or methacrylate polymer. A water soluble flavouring agent is dispersed within the inner polymer wall.

### US-A-1 996 203

Hollingsworth teaches a drinking straw having an interior which is coated with a soluble flavouring material so that when the straw is used the material will flavour the liquid being drawn through the straw (column 1, line 24 to column 2, line 7).

Nothing in these references or their combination teaches using a coating of a probiotic micro organism in any carrier, including in an oil or wax, nor use of an open bore with open ends containing a probiotic micro organism in any formulation.

### SUMMARY OF THE INVENTION

The invention herein is a dispensing tube containing living cells of a probiotic micro organism, such as bacterial cells or other additive, on the inside of the tube. The tube is wrapped and sealed in an outer watertight envelope until time for usage. At the time of usage the outer envelope is taken away and when the tubular device penetrates a solution container such as a beverage or an enteral solution, the living cells of a probiotic microorganism is added in the desired amount to the solution while the solution flows through the tube.

Other objects and features of the inventions will be more fully apparent from the following disclosure and appended claims.

### BRIEF DESCRIPTION OF THE DRAWINGS

Figure 1 is a perspective view of a first embodiment of the tube of the invention.
Figure 2 is a perspective view of a second embodiment of the tube of the invention.
Figure 3 is a perspective view of a third embodiment of the tube of the invention
Figure 4 is a cross-section of a portion of the tube that has an adherent the living cells of a probiotic microorganism inside the tube.
Figure 5 is a partial view of a partially sectioned tube end showing the location of adherent living cells of a probiotic microorganism.
Figures 6A-6C depict a coated second tube prior to insertion in the impermeable tube (Figure 6A); the coated second tube inserted partway into the impermeable tube (Figure 6B); and the rotation of the coated second tube end and dislodgment of the living cells of a probiotic microorganism inside the impermeable tube.
Figure 7 is a perspective view of the device of the invention packaged in an envelope.

### DETAILED DESCRIPTION OF THE INVENTION AND PREFERRED EMBODIMENTS THEREOF

The present invention is a dispensing tube having a structure similar to that of a drinking straw. The dispensing tube is impermeable to liquids and open at both ends for the purpose of delivering the solution through the device normally by sucking.

A suspension of typically probiotic microorganism, for example, lactic bacteria or bifidus bacteria, is added to the dispensing tube as described below. In the preferred embodiments of the invention, during production of the tube of the invention, a second tube delivers the the living cells of a probiotic microorganism into the dispensing tube. The tube is then placed into a water-resistant outer envelope using a packaging machine as is known in the art. The materials used in the manufacture of the invention, in particular, the dispensing tube and the outer envelope, must be capable of protecting the living cells of a probiotic microorganism from contamination and moisture for periods of up to 12 months storage at room temperature, The tube material must also be able to withstand the suspension media used for the the living cells of a probiotic microorganism

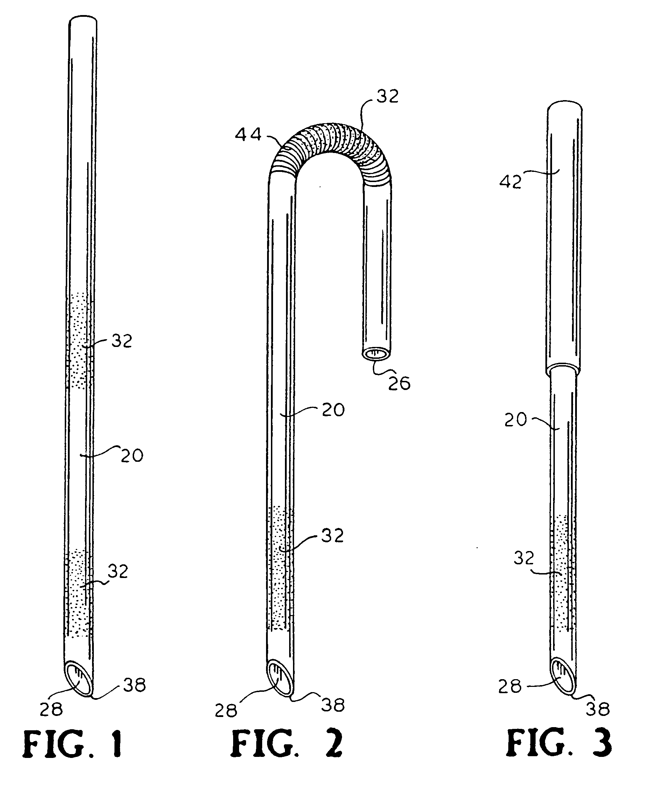

Referring to the figures, the invention herein is a dispensing tube for dispensing a the living cells of a probiotic microorganism into a liquid, comprising a liquid impermeable tube 20 having an open bore 22 surrounded by an inner tube wall 24. Bore 22 is open at both an upper end 26 and a lower end 28 of the tube as shown in **Figures 1-3**; in other words, the tube is not closed at either end. Tube 20 is preferably of a size and structure as is known with drinking straws, such as the straws that are used with boxed individual drink cartons, and is preferably formed from a synthetic polymeric material such as polyethylene or polypropylene, or from paper with an internal coating of a wax material, Preferred dimensions range from a width of 0.2-20 mm and a length of 50-500 mm. The tube may be a commercial drinking straw.

Thus, if tube 20 is to be used with a standard drink box having a puncturable opening, the lower end 28 of the tube is preferably a pointed end as shown in **Figures 1-3** and **7**, Other dispensing tubes that may be used in the invention include those with bellows 44 as is known in the art (**Figure 2**). Since straws used in Japan often have an outer tube into which the drinking straw telescopes, the invention herein also include an embodiment having an outer tube 42 as shown in **Figure 3**.

It is important that tube 20 have the capability to hold the suspension, and to hinder the suspension from unintentional leakage out of the tube 20. This is accomplished either by surface tension through appropriate selection of the material of which the tube is made or by treatment of tube 20. It also could be done by altering the viscosity of the suspension. Although not required for the invention, in the preferred embodiments of the invention, the inner wall of the tube is coated or is otherwise surface- modified to give a higher surface tension. Thus, in the preferred embodiment, the invention further includes a coating material 30 for holding a suspension of the the living cells of a probiotic microorganism 32 within the open bore 22 adherent to the inner tube wall 24. This coating material 30 may comprise any coating substance which is nontoxic to humans and to the bacteria to be added to the tube, and which adheres to the inner tube wall 24, for example, a dietary oil such as corn oil or a wax. The coating material may be applied in a number of ways, for example, by insertion of an elongated stick or other device coated with the substance, with a tubular filling device, or by spraying the inside of tube 20.

The bacteria or additive may be applied with the coating substance, or be separately applied to selected positions inside the tube (for example, a small drop of about 10 µl) evenly sprayed all over the inside of the tube after coating the inside of tube 20. The pattern and extent of distribution of the additive inside tube 20 may be determined by the nature of the additive and the intended use. Thus, for an easily soluble additive, even distribution is less critical. If the additive dissolves quickly and is at the distal end of tube 20, it may be desirable to position the additive close to the distal end, so that the additive becomes quickly dispersed in the solution in the container. Another reason to position the additive at the distal end is when the additive has an unpleasant taste, and it is desired to maximally dilute the additive before it is consumed. Alternatively, when the additive has a pleasant taste, it may be positioned at the upper end to increase the good taste of the drink. To minimize exposure of an oxygen-sensitive additive, the additive may be placed in the inside tube 20 in the form of a droplet, which would have less surface area than an evenly dispersed material inside the tube 20.

Preferably the the living cells of a probiotic microorganism 32 that is to be dispensed from the dispensing tube 20 comprises lyophilized cells of one or more probiotic microorganisms, such as various *Lactobacillus* or *Bifidobacteria* strains. Depending on the type of additive being added to tube 20, the suspension containing the additive may need to specially treated to optimize shelf stability and appropriate retention in, and release from, tube 20. For bacteria, the suspension containing the bacteria should not contain a significant amount of water, and should be fairly resistant to oxidation. The suspension should dissolve or release or carry the additive, such as bacteria, into the fluid that is being consumed, at typical use temperatures (e.g., 0°C-40°C). Thus, for a number of additives such as bacterial additives, the dried additive is preferably granulized into a very fine powder to insure solubility and even distribution in the solution.

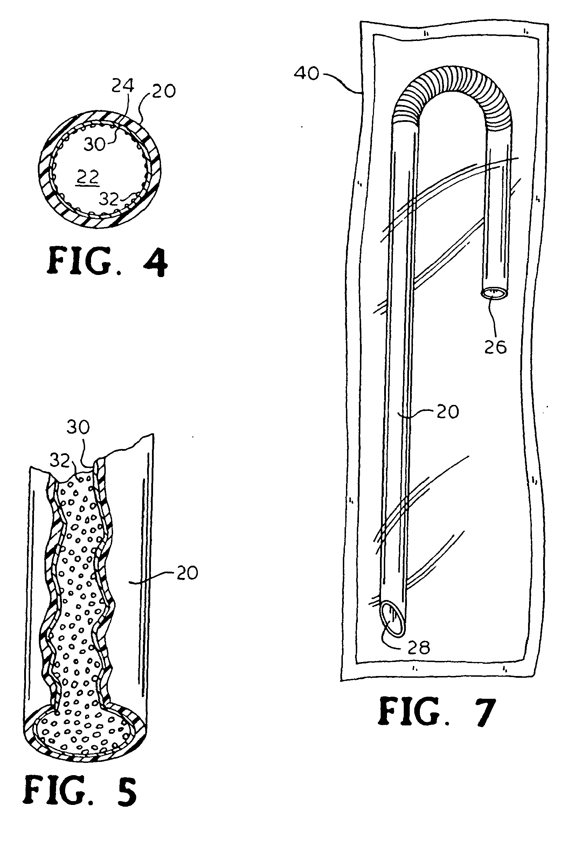

The bacterial suspension or other additive to be contained in the device is preferably prepared in a sufficiently concentrated formulation so that surface tension/adhesion withholds the suspension in the tube. The concentration of the suspension is optimized to give a good ratio between volume and number of bacteria per ml. Preferably the concentration of bacteria is not be lower then 1% in the suspension. The cells are preferably mixed directly into the suspending liquid under a nitrogen protective flow to reduce vapor and oxygen presence. Although cells of bacteria would not be visible to the naked eye, the the living cells of a probiotic microorganism 32 is depicted in the figures as small dots, or as small circles, to show their position (e.g., in Figures 1-4 and **Figure 5,** respectively).

Other selected materials that could be added to liquids using the device of the invention include vitamins, colorants, minerals, trace elements, homeopathic medicines, drugs, enzymes and the like.

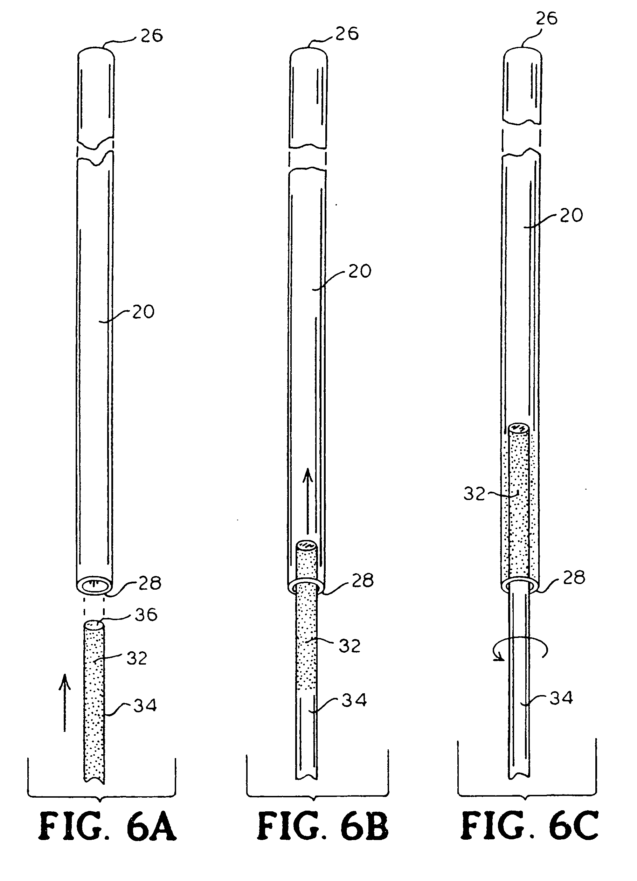

The selected living cells of a probiotic microorganism 32, such as bacterial cells, is preferably added to the tube 20 by providing a second tube 34 having a smaller outer diameter than the inner diameter of the liquid impermeable tube and preferably having a closed end 36 as shown in **Figures 6A-6C.** The closed end 36 is coated externally with the 32 as shown in **Figure 6A**, which adhere by surface tension and/or by use of an adhesive material as is known in the art. Then the coated closed end 36 is inserted into the bore 22 at the lower end 28 of the liquid impermeable tube 20 which has been treated in part or entirely with the coating material 30 (**Figure 6B**). The closed end 36 is moved, such as being rotated, against the coating material, thereby dislodging living cells of a probiotic microorganism that then adheres to the coating on the inner wall (**Figure 6C**).

The tube 20 is preferably packaged in a flexible, essentially water vapor tight envelope 40 enclosing the tube 20 as shown in **Figure 7**. The outer envelope must be substantially impermeable to water vapor and should have sufficient flexibility and toughness to prevent unintentional puncture, and is preferably made of flexible polymeric material or an aluminum foil, coated on a polymeric film. In all cases the envelope construction is made in such a way that when sealed, water and moisture are prevented from entering the tube device. The material should also be easy to tear open at the point of use. The envelope 40 is preferably made of a polyolefinic material coated with aluminum or of a synthetic polymer as is known in the art with a low water permeation rate. A preferred material for the outer envelope is a polyethylene or polypropylene, including both homopolymers and copolymers of these polymer families, with an aluminum layer as an outer layer. If a transparent envelope 40 is desired, the polyethylene/polypropylene structure may have outer layer comprising a polymer of ethyl vinyl alcohol or poly-vinylidene chloride. Alternatively, polyethylene and/or polypropylene may be used without an aluminum layer if there is not a need for protection from oxygen. Those of ordinary skill in the art may substitute other suitable packaging material.

To use the impermeable tube 20, it is removed from envelope 40, and inserted into a chosen liquid container by either lowering it through an opening in the container as is done with standard straws, or by puncturing a puncturable port on the container as is done with juice cartons. The port of the container, such as a juice box, to which the bacteria are to be added (not shown) may be protected by for instance a puncturable aluminum foil as is known in the art that will make it possible to add bacteria to an aseptic filled or a thermally sterilized solution. At the time of the straw's penetration of the container, for example, of an enteral product, a dairy product, a soft drink or some other type of solution or mixture, the bacteria are integrated into the solution, giving a desirable dose of bacteria in the product. Once the lower end 28 of the tube is immersed in the liquid and the living cells of a probiotic microorganism 32 is removed from the inner tube wall 24 and mixed with the liquid by drawing the liquid through the bore from the lower end to the upper end and into the mouth.

The envelope 40 containing the tube 20 can be sold separately from the beverage or other fluid containers, or can be attached to the container, for example, by adhesives as is known in the art for drinking container straws. Thus, tube 20 containing the living cells of a probiotic microorganism and in envelope 40 could easily be attached onto every type of package containing solutions where addition of the living cells of a probiotic microorganism would be suitable.

While the invention has been described with reference to specific embodiments, it will be appreciated that numerous variations, modifications, and embodiments are possible within the scope of the claims.

## Claims

1. A device for dispensing living cells of a probiotic microorganism into a liquid, comprising:
(a) a liquid impermeable dispensing tube (20) having an open bore (22) being surrounded by an inner tube wall (24) and extending from an open upper end (26) of the tube (20) to an open lower end (28) of the tube (20);
(b) a coating material (30) on a portion of the inner tube wall (24), said coating material (30) selected from the group consisting of oils and waxes and holding a suspension of the living cells of the probiotic microorganism for long-term storage within the open bore (22) adherent to the inner tube wall (24); and
(c) a flexible, essentially water vapour tight envelope (40) enclosing the tube (20) for long-term storage and to protect the living cells of the probiotic microorganism from moisture;
the device being adapted so that the living cells of the probiotic microorganism may be removed from the inner tube wall (24) and mixed with the liquid by placing the open lower end (28) of the tube (20) in the liquid and drawing the liquid through the bore (22).

2. The device of claim 1, wherein the probiotic microorganism comprises Lactobacillus.

3. The device of claim 1, wherein the cells of a probiotic microorganism comprise a suspension of lyophilized live bacteria.

4. The device of claim 3, in which the concentration of live bacteria at the point of use is at least 1% of the suspension when the device is manufactured.

5. The device of claim 1, wherein the viscosity of the suspension is modified to provide maximum adherence of the suspension to the device.

6. The device of claim 1, wherein the tube (20) comprises a bellows portion.

7. The device of claim 1, wherein the tube (20) telescopes into an outer tube (42).

8. The device of claim 1, wherein the tube (20) is made from a synthetic polymer material.

9. The device of claim 1, wherein the tube (20) is made from paper with an internal coating of a wax material.

10. The device of claim 1, wherein the tube (20) has a diameter ranging from 0.2-20 mm and a length of 50-500 mm.

11. The device of claim 1, wherein the tube (20) is a commercial drinking straw.

12. The device of claim 1, wherein the open bore (22) of the tube (20) has been surface modified to give a higher surface tension.

13. The device of claim 1, wherein the envelope is made of a polyolefinic material coated with aluminum.

14. The device of claim 1, wherein the envelope is made of a synthetic polymer with a low water permeation rate.

15. The device of claim 1, wherein the suspension of cells is distributed along the full length of the inner tube wall (24).

16. The device of claim 1, wherein the suspension of cells is primarily placed at the lower end of the tube (20).

17. The device of claim 1, wherein the coating material (30) comprises a dietary oil.

18. The device of claim 1, wherein the envelope comprises an aluminum layer.

19. A method of making a device for dispensing living cells of a probiotic microorganism into a liquid, comprising:
(a) providing a liquid impermeable dispensing tube (20) having an open bore (22) being surrounded by an inner tube wall (24) and extending from an open upper end (26) of the tube (20) to an open lower end (28) of the tube (20);
(b) coating a portion of the inner tube wall (24) within the open bore (22) with a coating material (30) selected from the group consisting of oils and waxes and holding a suspension of the living cells of the probiotic microorganism for long-term storage so that the coating material (30) containing the living cells adheres to the inner tube wall (24); and
(c) providing a flexible, essentially water vapour tight envelope (40) enclosing the tube (20) for long-term storage and to protect the living cells of the probiotic microorganism from moisture;
wherein the living cells of the probiotic microorganism may be removed from the inner tube wall (24) and mixed with the liquid by placing the open lower end (28) of the tube (20) in the liquid and drawing the liquid through the bore (22).

20. The method of claim 19, wherein the inner tube wall (24) is coated with the cells of the probiotic microorganism by insertion of a second tube (34) that has a coating of the cells of the probiotic microorganism into the lower end (28) of the dispensing tube (20).

21. The method of claim 19, wherein the probiotic microorganism comprises Lactobacillus.

22. The method of claim 19, wherein the cells of the probiotic microorganism comprise a suspension of lyophilized live bacteria.

23. The method of claim 22, in which the concentration of live bacteria at the point of use is at least 1% of the suspension when the device is manufactured.

24. The method of claim 19, wherein providing the liquid impermeable dispensing tube (20 comprises providing a tube (20) comprising a bellows portion.

25. The method of claim 19, wherein providing the liquid impermeable dispensing tube (20) comprises providing a tube (20) that telescopes into an outer tube (42).

26. The method of claim 19, wherein providing the liquid impermeable dispensing tube (20) comprises providing a tube (20) made from a synthetic polymer material.

27. The method of claim 19, wherein providing the liquid impermeable dispensing tube (20) comprises providing a tube (20) made from paper with an internal coating of a wax material.

28. The method of claim 19, wherein providing a liquid impermeable dispensing tube (20) comprises providing a tube (20) having a diameter ranging from 0.2-20 mm and a length of 50-500 mm.

29. The method of claim 19, wherein providing a liquid impermeable dispensing tube (20) comprises providing a commercial drinking straw.

30. The method of claim 19, further comprising surface-modification of the open bore (22) of the tube (20) to give a higher surface tension.

31. The method of claim 19, wherein providing the flexible, essentially water vapour tight envelope enclosing the tube (20) comprises providing a polyolefinic material coated with aluminum.

32. The method of claim 19, wherein providing the flexible, essentially water vapour tight envelope enclosing the tube (20) comprises providing an envelope made of a synthetic polymer with a low water permeation rate.

33. The method of claim 19, wherein coating a portion of the inner tube wall (24) comprises distributing the cells along the full length of the inner tube wall (24).

34. The method of claim 19, wherein coating a portion of the inner tube wall (24) comprises distributing the suspension of cells at the lower end (28) of the tube (20).

35. The method of claim 19, further comprising modifying the viscosity of the suspension to provide maximum adherence of the suspension to the device.

36. The method of claim 19, wherein the coating material comprises a dietary oil.

37. The method of claim 19, wherein the envelope comprises an aluminum layer.

## Patentansprüche

1. Vorrichtung zur Abgabe von lebenden Zellen eines probiotischen Mikroorganismus in eine Flüssigkeit, enthaltend:
(a) ein flüssigkeitsundurchlässiges Spenderrohr (20) mit einer offenen Bohrung (22) umgeben von einer inneren Rohrwand (24), die sich von einem oberen offenen Ende (26) des Rohrs (20) bis zu einem unteren offenen Ende (28) des Rohrs (20) erstreckt;
(b) ein Beschichtungsmaterial (30) auf einem Teil der inneren Rohrwand (24) des Beschichtungsmaterials (30), wobei das Beschichtungsmaterial aus der aus Ölen und Wachsen bestehenden Gruppe ausgewählt ist und eine Suspension von lebenden Zellen des probiotischen Mikroorganismus für Langzeitfagerung in der offenen Bohrung (22), die an der inneren Rohrwand (24) anhaftet, hält; und
(c) eine flexible, im wesentlichen wasserdampfdichte Umhüllung (40), die das Rohr (20) für Langzeitlagerung und zum Schutz der lebenden Zellen des probiotischen Mikroorganismus vor Feuchtigkeit einschließt;
wobei die Vorrichtung so angepasst ist, dass die lebenden Zellen des probiotischen Mikroorganismus von der inneren Rohrwand (24) entnommen und mit der Flüssigkeit gemischt werden können, indem man das offene untere Ende (28) des Rohrs (20) in die Flüssigkeit verbringt und die Flüssigkeit durch die Bohrung (22) saugt.

2. Vorrichtung nach Anspruch 1, wobei der probiotische Mikroorganismus Laktobazillus enthält.

3. Vorrichtung nach Anspruch 1, wobei die Zellen eines probiotischen Mikroorganismus eine Suspension von gefriergetrockneten lebenden Bakterien enthält.

4. Vorrichtung nach Anspruch 3, wobei die Konzentration von lebenden Bakterien am Ort der Anwendung mit wenigstens 1 % der Suspension ist, wenn die Vorrichtung hergestellt wird.

5. Vorrichtung nach Anspruch 1, wobei die Viskosität der Suspension so modifiziert wird, dass sie ein maximales Anhaften der Suspension an der Vorrichtung ergibt.

6. Vorrichtung nach Anspruch 1, wobei das Rohr (20) einen Balgteil aufweist.

7. Vorrichtung nach Anspruch 1, wobei das Rohr (20) teleskopartig in ein äußeres Rohr (42) hineinreicht.

8. Vorrichtung nach Anspruch 1, wobei das Rohr (20) aus einem synthetischen polymeren Material gemacht ist.

9. Vorrichtung nach Anspruch 1, wobei das Rohr (20) aus Papier mit einer inneren Beschichtung eines Wachsmaterials gemacht ist.

10. Vorrichtung nach Anspruch 1, wobei das Rohr (20) einen Durchmesser von 0,2-20 mm und eine Länge von 50-500 mm besitzt.

11. Vorrichtung nach Anspruch 1, wobei das Rohr (20) ein kommerzieller Trinkstrohhalm ist.

12. Vorrichtung nach Anspruch 1, wobei die offene Bohrung (22) des Rohrs (20) Oberflächen-modifiziert worden ist zur Erzeugung einer höheren Oberflächenspannung.

13. Vorrichtung nach Anspruch 1, wobei die Hülle aus einem mit Aluminium beschichteten polyolefinischen Material hergestellt ist.

14. Vorrichtung nach Anspruch 1, wobei die Hülle aus einem synthetischen Polymer mit niedriger Wasserdurchlässigkeitsrate gemacht ist.

15. Vorrichtung nach Anspruch 1, wobei die Zellsuspension über die ganze Länge der inneren Rohrwand (24) verteilt ist.

16. Vorrichtung nach Anspruch 1, wobei die Zellsuspension hauptsächlich am unteren Ende des Rohrs (20) angeordnet ist.

17. Vorrichtung nach Anspruch 1, wobei das Beschichtungsmaterial (30) ein diätetisches Öl enthält.

18. Vorrichtung nach Anspruch 1, wobei die Hülle eine Aluminiumschicht enthält.

19. Verfahren zur Herstellung einer Vorrichtung zur Abgabe von lebenden Zellen eines probiotischen Mikroorganismus in eine Flüssigkeit, umfassend:
(a) man beschafft ein flüssigkeitsundurchlässiges Spenderrohr (20) mit einer offenen Bohrung (22), die von einer inneren Rohrwand (24) umgeben ist und sich von einem oberen Ende (26) des Rohrs (20) bis an ein offenes unteres Ende (28) des Rohrs (20) erstreckt;
(b) man beschichtet einen Teil der inneren Rohrwand (24) innerhalb der offenen Bohrung (22) mit einem Beschichtungsmaterial (30) ausgewählt aus der aus Ölen und Wachsen bestehenden Gruppe, das eine Suspension der lebenden Zellen des probiotischen Mikroorganismus zur Langzeitlagerung enthält, so dass das Beschichtungsmaterial (30), das die lebenden Zellen enthält, an der inneren Rohrwand (24) haftet; und
(c) man beschafft eine flexible, im wesentlichen wasserdichte Hülle (40), die das Rohr (20) umschließt, zur Langzeitiagerung und zum Schutz der lebenden Zellen des probiotischen Mikroorganismus vor Feuchtigkeit; wobei die lebenden Zellen des probiotischen Mikroorganismus von der inneren Rohrwand (24) entfernt und mit der Flüssigkeit gemischt werden können, indem man das offene untere Ende (28) des Rohrs (20) in die Flüssigkeit bringt und die Flüssigkeit durch die Bohrung (22) saugt.

20. Verfahren nach Anspruch 19, wobei die innere Rohrwand (24) mit den Zellen des probiotischen Mikroorganismus beschichtet wird, indem man ein zweites Rohr (34), das eine Beschichtung der Zellen des probiotischen Mikroorganismus hat, in das untere Ende (28) des Spenderrohrs (20) einführt.

21. Verfahren nach Anspruch 19, wobei der probiotische Mikroorganismus Laktobazillus enthält

22. Verfahren nach Anspruch 19, wobei die Zellen des probiotischen Mikroorganismus eine Suspension von gefriergetrockneten lebenden Bakterien enthält.

23. Verfahren nach Anspruch 22, wobei die Konzentration von lebenden Bakterien am Ort der Anwendung wenigstens 1 % der Suspension ist, wenn die Vorrichtung hergestellt wird.

24. Verfahren nach Anspruch 19, wobei das Schaffen des flüssigkeitsundurchlässigen Abgaberohrs (20) die Schaffung eines Rohrs (20), das einen Balganteil aufweist, umfasst.

25. Verfahren nach Anspruch 19, wobei die Beschaffung des flüssigkeitsundurchlässigen Abgaberohrs (20) die Schaffung eines Rohrs (20) umfasst, das in ein äußeres Rohr (42) teleskopartig hineinreicht.

26. Verfahren nach Anspruch 19, wobei die Schaffung des flüssigkeitsundurchlässigen Abgaberohrs (20) die Schaffung eines Rohrs (20) aus einem synthetischen Polymermaterial umfasst.

27. Verfahren nach Anspruch 19, wobei das Beschaffen des flüssigkeitsundurchlässigen Abgaberohrs (20) die Beschaffung eines Rohrs (20), hergestellt aus Papier mit einer inneren Beschichtung von einem Wachsmaterial umfasst.

28. Verfahren nach Anspruch 19, wobei das Beschaffen eines flüssigkeitsundurchlässigen Abgaberohrs (20) das Beschaffen eines Rohrs (20) mit einem Durchmesser von 0,2-20 mm und einer Länge von 50-500 mm umfasst.

29. Verfahren nach Anspruch 19, wobei das Beschaffen eines flüssigkeitsundurchlässigen Abgaberohrs (20) das Beschaffen eines handelsüblichen Trinkstrohhalms umfasst.

30. Verfahren nach Anspruch 19, weiterhin umfassend eine Oberflächenmodifizierung der offenen Bohrung (22) des Rohrs (20) zur Erzeugung einer höheren Oberflächenspannung.

31. Verfahren nach Anspruch 19, wobei das Beschaffen der flexiblen, im wesentlichen wasserdampfdichten Hülle, die das Rohr (20) einschließt, die Beschaffung eines mit Aluminium beschichteten Polyolefinmaterials umfasst.

32. Verfahren nach Anspruch 19, wobei die Beschaffung der flexiblen, im wesentlichen wasserdampfdichten Hülle, die das Rohr (20) einschließt, die Beschaffung einer Hülle aus einem synthetischen Polymer mit einer niedrigen Wasserdurchlässigkeitsrate umfasst.

33. Verfahren nach Anspruch 19, wobei die Beschichtung der inneren Rohrwand (24) die Verteilung der Zellen über die Gesamtlänge der inneren Rohrwand (24) umfasst.

34. Verfahren nach Anspruch 19, wobei das Beschichten eines Teils der inneren Rohrwand (24) die Verteilung der Zellsuspension am unteren Ende (28) des Rohrs (20) umfasst.

35. Verfahren nach Anspruch 19, weiterhin umfassend die Modifizierung der Viskosität der Suspension zur Erzeugung einer maximalen Haftung der Suspension an der Vorrichtung.

36. Verfahren nach Anspruch 19, wobei das Beschichtungsmaterial ein diätetisches Öl umfasst,

37. Verfahren nach Anspruch 19, wobei die Hülle eine Aluminiumschicht umfasst.

## Revendications

1. Dispositif de distribution de cellules vivantes d'un micro-organisme probiotique dans un liquide, comprenant :
(a) un tube de distribution imperméable aux liquides (20) comportant un canal ouvert (22) entouré par une paroi intérieure du tube (24) et s'étendant d'une extrémité supérieure ouverte (26) du tube (20) à une extrémité inférieure ouverte (28) du tube (20) ;
(b) un matériau de revêtement (30) sur une portion de la paroi intérieure du tube (24), ledit matériau de revêtement (30) étant choisi dans le groupe constitué par des huiles et des cires et conservant une suspension de cellules vivantes du micro-organisme probiotique adhérant à la paroi intérieure du tube (24) en vue d'un stockage à long terme à l'intérieur du canal ouvert (22) ; et
(c) une enveloppe souple (40), sensiblement étanche à la vapeur d'eau, enfermant le tube (20) en vue d'un stockage à long terme et pour protéger les cellules vivantes du micro-organisme probiotique contre l'humidité ;
le dispositif étant adapté de telle sorte que les cellules vivantes du micro-organisme probiotique peuvent être retirées de la paroi intérieure du tube (24) et mélangées au liquide en plaçant l'extrémité inférieure ouverte (28) du tube (20) dans le liquide et en aspirant le liquide par le canal (22).

2. Dispositif selon la revendication 1 dans lequel le micro-organisme probiotique inclut Lactobacillus.

3. Dispositif selon la revendication 1, dans lequel les cellules d'un micro-organisme probiotique incluent une suspension de bactéries vivantes lyophilisées.

4. Dispositif selon la revendication 3, dans lequel la concentration en bactéries vivantes au moment de l'utilisation est d'au moins 1% de la suspension lors de la fabrication du dispositif.

5. Dispositif selon la revendication 1, dans lequel la viscosité de la suspension est modifiée pour conférer une adhérence maximale de la suspension au dispositif.

6. Dispositif selon la revendication 1, dans lequel le tube (20) comprend une portion à soufflet.

7. Dispositif selon la revendication 1, dans lequel le tube (20) pénètre de façon télescopique dans un tube extérieur (42).

8. Dispositif selon la revendication 1, dans lequel le tube (20) est en un matériau polymère synthétique.

9. Dispositif selon la revendication 1, dans lequel le tube (20) est en carton doté d'un revêtement intérieur de matière cireuse.

10. Dispositif selon la revendication 1, dans lequel le tube (20) a un diamètre allant de 0,2 à 20 mm et une longueur allant de 50 à 500 mm.

11. Dispositif selon la revendication 1, dans lequel le tube (20) est une paille pour boire du commerce.

12. Dispositif selon la revendication 1, dans lequel le canal ouvert (22) du tube (20) a été modifié en surface pour conférer une tension superficielle plus élevée.

13. Dispositif selon la revendication 1, dans lequel l'enveloppe est en un matériau polyoléfinique revêtu d'aluminium.

14. Dispositif selon la revendication 1, dans lequel l'enveloppe est en un polymère synthétique avec un faible taux de pénétration de l'eau.

15. Dispositif selon la revendication 1, dans lequel la suspension de cellules est distribuée sur toute la longueur de la paroi intérieure du tube (24).

16. Dispositif selon la revendication 1, dans lequel la suspension de cellules est essentiellement placée à l'extrémité inférieure du tube (20).

17. Dispositif selon la revendication 1, dans lequel le matériau de revêtement (30) inclut une huile alimentaire.

18. Dispositif selon la revendication 1, dans lequel l'enveloppe inclut une couche d'aluminium.

19. Procédé de fabrication d'un dispositif de distribution de cellules vivantes d'un micro-organisme probiotique dans un liquide, comprenant les étapes consistant à :
(a) fournir un tube de distribution imperméable aux liquides (20) comportant un canal ouvert (22) entouré par une paroi intérieur du tube (24) et s'étendant d'une extrémité supérieure ouverte (26) du tube (20) à une extrémité inférieure ouverte (28) du tube (20) ;
(b) enduire une portion de la paroi intérieure du tube (24) à l'intérieur du canal ouvert (22) avec un matériau de revêtement (30) choisi dans le groupe constitué par des huiles et des cires et conservant une suspension de cellules vivantes du micro-organisme probiotique en vue d'un stockage à long terme de telle sorte que le matériau de revêtement contenant les cellules vivantes adhère à la paroi intérieure du tube (24) ; et
(c) fournir une enveloppe souple (40), sensiblement étanche à la vapeur d'eau, enfermant le tube (20) en vue d'un stockage à long terme et pour protéger les cellules vivantes du micro-organisme probiotique contre l'humidité ;
dans lequel les cellules vivantes du micro-organisme probiotique peuvent être retirées de la paroi intérieure du tube (24) et mélangées au liquide en plaçant l'extrémité inférieure ouverte (28) du tube (20) dans le liquide et en aspirant le liquide à travers le canal (22).

20. Procédé selon la revendication 19, dans lequel la paroi intérieure du tube (24) est recouverte avec les cellules du micro-organisme probiotique en insérant un deuxième tube (34) qui est doté d'un revêtement de cellules du micro-organisme probiotique dans l'extrémité inférieure (28) du tube de distribution (20).

21. Procédé selon la revendication 19, dans lequel le micro-organisme probiotique inclut Lactobacillus.

22. Procédé selon la revendication 19, dans lequel les cellules du micro-organisme probiotique incluent une suspension de bactéries vivantes lyophilisées.

23. Procédé selon la revendication 22, dans lequel la concentration en bactéries vivantes au moment de l'utilisation est d'au moins 1% de la suspension lors de la fabrication du dispositif.

24. Procédé selon la revendication 19, dans lequel la fourniture du tube de distribution imperméable aux liquides (20) inclut la fourniture d'un tube (20) comprenant une portion à soufflet.

25. Procédé selon la revendication 19, dans lequel la fourniture du tube de distribution imperméable aux liquides (20) inclut la fourniture d'un tube (20) qui pénètre de façon télescopique dans un tube extérieur (42).

26. Procédé selon la revendication 19, dans lequel la fourniture du tube de distribution imperméable aux liquides (20) comprend la fourniture d'un tube (20) en matériau polymère synthétique.

27. Procédé selon la revendication 19, dans lequel la fourniture du tube de distribution imperméable aux liquides (20) inclut la fourniture d'un tube (20) en carton doté d'un revêtement intérieur en matière cireuse.

28. Procédé selon la revendication 19, dans lequel la fourniture d'un tube de distribution imperméable aux liquides (20) inclut la fourniture d'un tube (20) ayant un diamètre de l'ordre de 0,2 à 20 mm et une longueur de 50 à 500 mm.

29. Procédé selon la revendication 19, dans lequel la fourniture d'un tube de distribution imperméable aux liquides (20) inclut la fourniture d'une paille pour boire du commerce.

30. Procédé selon la revendication 19, comprenant en outre une modification de surface du canal ouvert (22) du tube (20) pour conférer une tension superficielle plus élevée.

31. Procédé selon la revendication 19, dans lequel la fourniture de l'enveloppe souple, sensiblement étanche à la vapeur d'eau enfermant le tube (20), comprend la fourniture d'un matériau polyoléfinique recouvert d'aluminium.

32. Procédé selon la revendication 19, dans lequel la fourniture de l'enveloppe souple, sensiblement étanche à la vapeur d'eau enfermant le tube (20), comprend la fourniture d'une enveloppe en polymère synthétique à faible taux de pénétration de l'eau.

33. Procédé selon la revendication 19, dans lequel le revêtement d'une portion de la paroi intérieure du tube (24) comprend la distribution des cellules sur toute la longueur de la paroi de tube intérieure (24).

34. Procédé selon la revendication 19, dans lequel le revêtement d'une portion de la paroi intérieure du tube (24) comprend la distribution de la suspension de cellules à l'extrémité inférieure (28) du tube (20).

35. Procédé selon la revendication 19, comprenant en outre la modification de la viscosité de la suspension pour conférer une adhérence maximale de la suspension au dispositif.

36. Procédé selon la revendication 19, dans lequel le matériau de revêtement inclut une huile alimentaire.

37. Procédé selon la revendication 19, dans lequel l'enveloppe inclut une couche d'aluminium.
